Europäisches Patentamt

European Patent Office

Office européen des brevets

(11) Publication number: **0 407 368 B1**

# EUROPEAN PATENT SPECIFICATION

(45) Date of publication of patent specification: **12.10.94** (51) Int. Cl.⁵: **G01M 3/40, //A61B19/04**

(21) Application number: **90850259.4**

(22) Date of filing: **03.07.90**

(54) **Apparatus and method for detecting leaks in surgical gloves.**

(30) Priority: **05.07.89 US 376065**

(43) Date of publication of application:
**09.01.91 Bulletin 91/02**

(45) Publication of the grant of the patent:
**12.10.94 Bulletin 94/41**

(84) Designated Contracting States:
**AT BE CH DE DK ES FR GB GR IT LI LU NL SE**

(56) References cited:
FR-A- 2 208 300
GB-A- 859 579
US-A- 2 981 886
US-A- 4 909 069

(73) Proprietor: **Leach, Eddie D.**
**627 Charlie Hicks Road**
**Jonesborough, Tennessee 37659 (US)**

(72) Inventor: **Leach, Eddie D.**
**627 Charlie Hicks Road**
**Jonesborough, Tennessee 37659 (US)**

(74) Representative: **Wiklund, Erik et al**
**AWAPATENT AB,**
**Box 5117**
**S-200 71 Malmö (SE)**

## Description

## BACKGROUND

The present invention relates to an apparatus and a method for detecting holes or perforations in a rubber or plastic surgical or examination glove according to the preamble of Claims 1 and 4 respectively.

Around the turn of the century, due in a large extent to work with bacteria began by Louis Pasteur, it became recognized that there is a need for protection against cross infection between patients and health care professionals, and subsequently between patients contacted by the health care professional. Because of this, many modern techniques have been developed to ensure disinfection and sterilization of instruments and equipment. Also to this end, barriers to microbes, such as gowns, face masks and surgical gloves are generally worn by the health care professional when potentially infectious conditions are encountered. More recently, because of the spread of AIDS and hepatitis, health care professionals wear such barriers much more routinely than they did in the past.

Surgical gloves do much to protect the health care professional from infection but are inconvenient and expensive if the gloves have to be changed between each patient. Fortunately, studies have shown that wearing surgical gloves, and washing the gloved hands between patients, is more effective in preventing cross-contamination between patients than is washing the hands, apparently because pores, wrinkles and the otherwise rough surface of the hands may sometimes protect trapped bacteria from being washed away.

Because of the problems encountered in mass manufacturing surgical gloves, there is always a risk that a new glove might have a leak or undetectable micro-tear. Presently, federal regulations permit 4 out of every 100 examination gloves to have pinholes. The use, washing, and reuse of gloves tends to raise the probability that a leak or tear may develop. If a non-obvious leak or tear is present in a surgical glove, the health care professional may be at risk without realizing the risk.

Two basic approaches to the problem of leak testing surgical gloves prior to use or when gloves that are in use are leak tested periodically are known. One approach typified by U.S. Patent No. 2,981,886 is to have the user stand on a conductive surface and immerse his gloved hands into a basin containing an electrolytic solution. A voltage is then applied across the conductive surface and the electrolytic solution to permit current flow through an indicator when a hole exists in the gloves.

The other approach typified by GB-A-859 579 and FR-A-2 208 300 is to immerse the gloved hands into a bowl of an electrolytic liquid, and apply a voltage between the liquid and a bared part of the user's body to permit current flow through an indicator when a hole exists in the gloves.

One problem with these prior art approaches is that cross-contamination may occur if the electrolytic liquid is used more than once for leak testing.

It is an object of the present invention to reduce or substantially eliminate the risk that a health care professional will become contaminated from an infectious patient while using a surgical glove that has a small leak, tear or pinhole which is not readily apparent.

Other objects of the invention will be readily apparent from the following description and claims.

## SUMMARY OF THE INVENTION

According to the present invention the objects are achieved by an apparatus and a method as set forth in the appended Claims.

## BRIEF DESCRIPTION OF THE DRAWING

FIG. 1 is a schematic illustration of one embodiment of the apparatus.

FIG. 2 is an illustration of the apparatus in use in one possible embodiment of the method of the invention.

FIG. 3 is a schematic illustration of a second embodiment of the apparatus of the invention.

## DETAILED DESCRIPTION OF THE INVENTION

The present invention is described in detail for its use in detecting leaks, tears and/or pinholes in surgical or examination gloves.

Most surgical or examination gloves are made of some type of latex rubber or plastic which acts as a dielectric. That is, it will not allow the passage of ions which are driven by a given voltage unless there is a tear or perforation in the membrane making up the glove.

Ions are considerably smaller than the smallest of the known infectious agents, viruses. Bacteria are considerably larger than viruses. Therefore, if a glove is impervious to ions driven by an electric potential, then the glove should also be impervious to viruses and other infectious agents, which are many times larger than the ions, and are not driven by any electric potential. If ions can pass through a glove, then the glove should be judged defective and should be discarded, thus protecting both the patient and health care professional.

Stated briefly, the apparatus of the present invention includes a first electrically conductive lead having a first end indirectly contacting a fluid, such lead having a second end connected to a poker source, a second electrically conductive lead having a first end for directly contacting a conductive element which is adapted to substantially contact one surface of the glove which is being tested for perforations, and a second end attached to the power source, and an electrical measuring device for measuring the electrical properties between the first lead and the second lead when a leak in the article causes a completion of a circuit between the first lead and the second lead through the fluid.

The method of the present invention includes the steps of placing the article being tested for perforations on a conductive element that substantially fills the glove to provide a glove covered conductive element, connecting a first end of a first electrical lead to a power source and a second end of the lead to a fluid containing ions, immersing the outside surface of the glove covered conductive element in the fluid, connecting a first end of a second electrical lead directly to the glove covered conductive element and a second end of the lead to the power source, and observing the electrical properties between the second end of the first lead and the first end of the second lead using an electrical measuring device electrically connected to the first and second leads.

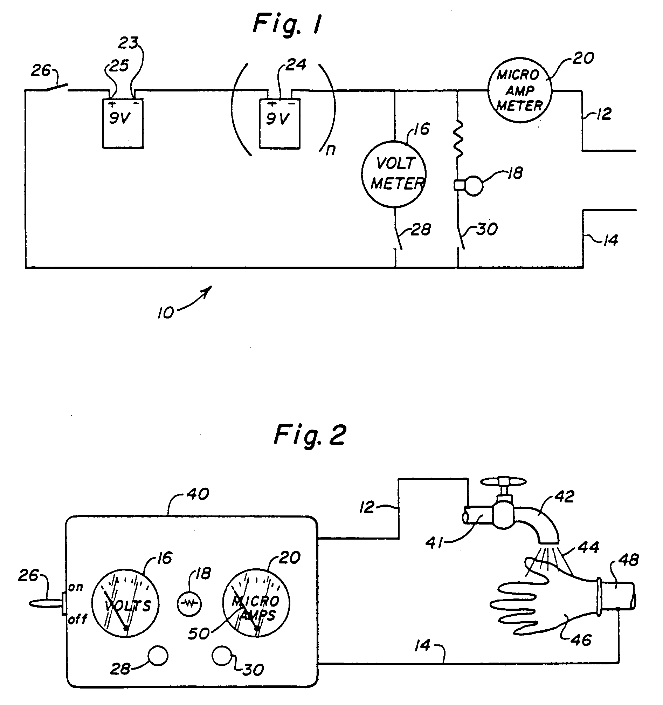

Referring now to FIG. 1, in a preferred embodiment, testing apparatus 10 of the invention comprises a first test lead 12 and a second test lead 14, test leads 12 and 14 being connected to opposite poles of battery power source 24. Battery power source 24 may comprise a number (n + 1) of 9 volt batteries connected in series, where n can be 0 to 14. In the illustrated embodiment lead 12 is connected to negative pole 23, and lead 14 is connected to positive pole 25. It has been found that the particular polarity of the leads does not affect the test procedure. The power source is controlled by on/off switch 26. In the preferred embodiment the power source is rechargeable batteries. Volt meter 16, which is used to test the batteries, is in series with the batteries when switch 28 is closed. Test lamp 18, which is used to test the circuit, is in series with the batteries when switch 30 is closed. A test lamp is desirable because when a negative result is obtained using the apparatus, i.e. when a glove is tested that has no leaks, the circuit will not be completed to activate microamp meter 20, and some means is needed in the circuit to provide assurance to the operator that the circuits are operating correctly.

Microammeter 20 will be in series with power source 24 when a leak develops in a glove and a circuit is completed during testing.

Since, for proper operation, it is desirable that switches 28 and 30 be open when testing a surgical glove, and switch 30 should be open when testing volt meter 16, switches 28 and 30 may be provided as push button switches that are closed only as long as the operator maintains pressure on the switch.

It has been demonstrated that gloves can be easily and effectively disinfected in a very short period of time, for example by washing the gloved hands before use. If this is done, and the glove checks to be safe through the use of the present invention, and the same procedures are followed before each use, then the same glove can be used in the treatment of several patients in a dental office, podiatrist, dermatologist, and other similar circumstances, where it is not practical to maintain operating room conditions.

With reference now to FIG. 2, an embodiment of the apparatus of the invention is illustrated in use in one possible method of using the invention. In the illustrated embodiment, the elements of the device are contained in cabinet 40. In the method, after testing the device to ensure that it is operating correctly, by first depressing switch 28 and then switch 30, test lead 12 is connected to a water pipe 41, or other electrical conductive portion of tap 42, and the tap is opened causing flow of water 44. The practitioner, while wearing surgical gloves, touches or has an assistant touch test lead 14 to his arm 48 above glove 46, and washes his hands under running water 44. If a leak, hole or tear is present in the glove, the ions in the tap water and/or in the soap used, carry a flow of electrons from power source 24 through water 44 to the hole or tear and into contact with the skin under the hole or tear. Since the skin conducts electricity, the skin and arm act as a conduit for carrying electrons to test lead 14 and back to power source 24. The flow of electrons through microammeter 20 causes the needle 50 to react and measure the amount of current produced.

Since the human body is sensitive to electrical shock, it is desirable that the current, $I = E/R$, be kept very low. The resistance of skin to electrical current is in the megaohm range, and accordingly, if the voltage of the power source is kept low, the current readings obtained will be in the microamp range, which produces an electron flow that is not detectable by most people. The high impedance, inherent resistance, and lower amperage of the output circuitry help prevent detection, by electrical shock, of the low current flow.

It will be recognized by those skilled in the art that the amplitude of the reading obtained may be variable, subject to the size of the hole (more ions may breach the opening) and the position of the hole (the length of the skin patch needed to complete the circuit, and the type of skin traversed may cause some variation in the resistance). In general, however, it can be demonstrated that the apparatus is sensitive enough to detect an opening the size of a sodium ion, or larger. Any leak (or pore) smaller than a sodium ion is not big enough for any known bacteria or virus to breach.

If the voltage of the power source is too small, the electromotive force (EMF) will not be great enough to drive the ions in the fluid to complete a circuit, and if the EMF is too high, the transfer of electrons through the glove may cause a hole in the glove where none existed before. Accordingly, the power source will provide about 5-90 volts, preferably 15-75 volts, and most preferably 20-50 volts. In the illustrated embodiment, the voltage provided is about 40 volts. A typical reading obtained on the microammeter when a leak is detected in a glove when tested on a hand is about 25-50 microamps.

Instead of measuring current, those skilled in the art will recognize that a leak also may be detected by measuring the potential (voltage) between the test leads, or the resistance between the test leads.

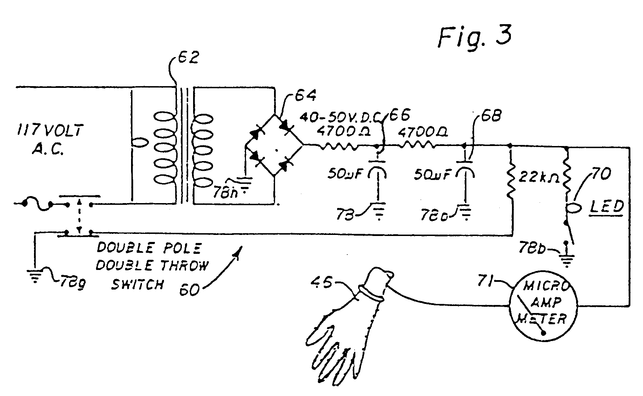

With reference now to FIG. 3, a schematic of an alternative embodiment of the apparatus 60 and a method is illustrated. The apparatus 60 is adapted to be connected to an A.C. (alternating current) power source, and since it is preferred that the current that contacts the skin be D.C. (direct current), apparatus 60 comprises (transformer) inductance coil 62, rectifier (filter circuit) 64, and capacitors 66 and 68 to convert A.C. to D.C. In the illustrated embodiment, a 120 volt primary to 24 volt secondary transformer is used to drive an A.C. to D.C. converter. Through proper filtering the pulsating D.C. is made into non-pulsating D.C. As illustrated, an LED 70 can be used to test the circuit and to provide a display means which lights to indicate the circuit is operating properly. A movement of the needle of the microammeter 71 indicates when a leak, hole, of (or) perforation is present in the article being tested.

The apparatus 60 is connected by one lead to the conductive portion of a water tap, the other lead to a body part above the gloved hand. The test procedure involves putting the gloved hand into the water flowing from the tap as described previously.

Yet another important feature of this method would be the use of sterile saline solution as a conductive medium in instances where maintaining sterile gloves and using aseptic technique is required as in a hospital operating room setting or other similar circumstances.

It has been found that about 1/4 teaspoon NaCl in 1 and 1/2 quarts of water provides a sufficient electrolyte solution for testing. Those skilled in the art will recognize that other electrolytes and other concentrations of electrolytes can be used.

If desired, a disinfectant may be added to the electrolyte solution. A suitable disinfectant is Expor™, available from Alcide Corp., Norwalk, Conn., which produces chlorine dioxide in solution. Household bleach can also be used. Other suitable disinfectants will be apparent to those skilled in the art.

Those skilled in the art will recognize that the apparatus can be used where the tested articles are made as a quality control device. For example, in a factory, an electrically conductive hand prosthesis, can be placed in the glove, or the glove can, be placed or formed over the hand pro(s)-thesis, and the glove can then be immersed in an electrolyte solution, and the test can be conducted otherwise as described above.

Those skilled in the art will recognize that the ion containing fluid, in certain embodiments, especially for factory testing, may be an ion containing gas.

Similarly, especially in a factory setting, instead of a hand, the conductive element which contacts one surface of the glove also may be an ion containing fluid.

While present embodiments of the invention and methods of practicing the same have been illustrated and described, it will be recognized by those skilled in the art treat this invention may be otherwise variously embodied and practiced within the scope of the following claims.

**Claims**

1. An apparatus for detecting holes or perforations in a rubber or plastic surgical or examination glove (46) having an inside surface and an outside surface with the latter being subject to contact with potentially infectious bacteria and viral agents during the use of said glove, said apparatus comprising:

   a first electrically conductive lead (12) having first and second ends with the first end indirectly contacting the outside surface of said glove (46) through a ion-containing liquid (44) when the outside surface of said glove is contacted by said liquid and with the second end connected to a power source (24);

   a second electrically conductive lead (14) having a first end for directly contacting a conductive element (48) which is adapted to substantially contact the inside surface of said glove and a second end attached to said power source;

   means (18, 20) for measuring the electrical properties between said first lead (12) and said second lead (14) when a leak through said glove allows ions in said liquid in contact with the outside surface of said glove to penetrate said leak and effect a completion of a circuit between said first lead and said second lead through said liquid, said means for measuring being electrically connected with said first lead and said second lead;

**characterized** in that the apparatus comprises liquid supply means (41) for providing a non-recirculating stream of the ion-containing liquid, the said first end of the first electrically conductive lead (12) being connected to the liquid supply means (41).

2. An apparatus for detecting holes or perforations in a rubber or plastic surgical or examination glove as claimed in claim 1, wherein said liquid supply means (41) provides a flow of tap water that defines said stream of ion-containing liquid, and wherein tap means (42) are operatively associated with said liquid supply means for controlling the flow of said tap water therefrom.

3. An apparatus for detecting holes or perforations in a rubber or plastic surgical or examination glove as claimed in claim 1, wherein said liquid supply means comprises container means for housing and providing a flow of sterile saline solution that defines said stream of ion-containing liquid.

4. A method for detecting a leak through a surgical or examination glove (46) formed of a substantially non-conductive material and having an inside surface and an outside surface with the latter surface being subject to contact with potentially infectious bacteria and viral agents during the use of said glove, said method comprising the steps of:

placing said glove on a conductive element (48) that substantially fills and contacts the inside surface of said glove to provide a glove-covered conductive element;

providing a ion-containing liquid;

connecting a first end of a first electrical lead (12) to the ion-containing liquid and a second end of said electrical lead to an electrical power source (24);

immersing the outside surface of said glove in said liquid for electrically coupling said first end of the first electrical lead to the outside surface of said glove;

connecting a first end of a second electrical lead (14) to said conductive element and a second end of said lead to said power source;

observing the electrical properties between the first end of said first lead and the first end of said second lead using an electrical measuring device (18, 20) electrically connected to said first and second leads for the detecting of a leak through the glove during the contact of the outside surface thereof by the liquid as determined by ions in said liquid penetrating said leak and completing an electrical circuit between said first and second leads;

**characterized** by the steps of providing the ion-containing liquid in the form of a stream of liquid;

connecting the first end of the first electrical lead (12) to the stream of liquid; and

disposing without recirculating the stream of liquid after contact with the outside surface of said glove.

5. A method for detecting a leak through a surgical or examination glove formed of a substantially non-conductive material as claimed in claim 4, wherein the stream of ion-containing liquid is provided by a flow of tap water from an open tap (42).

6. A method for detecting a leak through a surgical or examination glove formed of a substantially non-conductive material as claimed in claim 4, wherein the stream of ion-containing liquid is provided by a flow of sterile saline solution form a container.

**Patentansprüche**

1. Vorrichtung zur Feststellung von Löchern oder Poren in einem Operations- oder Untersuchungshandschuh (46) aus Gummi oder Kunststoff mit einer Innenseite und einer Außenseite, wobei letztere beim Einsatz des Handschuhs mit potentiell infektiösen Bakterien und Viruserregern in Kontakt kommt, wobei die Vorrichtung umfaßt:

eine erste elektrisch leitende Leitung (12) mit einem ersten und einem zweiten Ende, wobei das erste Ende über eine ionenhaltige Flüssigkeit (44) indirekt mit der Außenseite des Handschuhs (46) in Kontakt kommt, wenn die Außenseite des Handschuhs mit der Flüssigkeit in Kontakt kommt, und wobei das zweite Ende an eine Spannungsquelle (24) angeschlossen ist;

eine zweite elektrisch leitende Leitung (14) mit einem ersten Ende, das direkt mit einem leitenden Element (48) in Kontakt kommt, das im wesentlichen mit der Innenseite des Handschuhs in Kontakt kommt, sowie mit einem zweiten Ende, das an die Spannungsquelle angeschlossen ist;

Einrichtungen (18, 20) zum Messen der elektrischen Eigenschaften zwischen der ersten Leitung (12) und der zweiten Leitung (14), wenn es ein Loch in dem Handschuh Ionen in der Flüssigkeit, die mit der Außenseite des Handschuhs in Kontakt ist, ermöglicht, in das Loch einzudringen und über die Flüssigkeit einen Stromkreis zwischen der ersten Leitung und der zweiten Leitung zu schließen, wobei die Einrichtungen zum Messen elektrisch mit der ersten Leitung und der zweiten Leitung verbunden sind;
**dadurch gekennzeichnet**, daß die Vorrichtung eine Flüssigkeitszufuhreinrichtung (41) umfaßt, die einen nicht zirkulierenden Strom der ionenhaltigen Flüssigkeit erzeugt, wobei das erste Ende der ersten elektrisch leitenden Leitung (12) mit der Flüssigkeitszufuhreinrichtung (41) verbunden ist.

2. Vorrichtung zur Festellung von Löchern oder Poren in einem Operations- oder Untersuchungshandschuh aus Gummi oder Kunststoff nach Anspruch 1, wobei die Flüssigkeitszufuhreinrichtung (41) einen Leitungswasserstrom erzeugt, der den Strom ionenhaltiger Flüssigkeit bildet, und wobei eine Hahneinrichtung (42) funktionell mit der Flüssigkeitszufuhreinrichtung verbunden ist, um den Leitungswasserstrom aus selbiger zu regulieren.

3. Vorrichtung zur Feststellung von Löchern oder Poren in einem Operations- oder Untersuchungshandschuh aus Gummi oder Kunststoff nach Anspruch 1, wobei die Flüssigkeitszufuhreinrichtung eine Behältereinrichtung zur Aufnahme und zur Erzeugung eines Stroms keimfreier Kochsalzlösung umfaßt, der den Strom ionenhaltiger Flüssigkeit bildet.

4. Verfahren zur Feststellung eines Lochs in einem Operations- oder Untersuchungshandschuh (46), der aus einem im wesentlichen nichtleitenden Material besteht und eine Innenseite und eine Außenseite aufweist, wobei letztere Seite beim Einsatz des Handschuhs mit potentiell infektiösen Bakterien und Viruserregern in Kontakt kommt, wobei das Verfahren die folgenden Schritte umfaßt:
Anbringen des Handschuhs auf einem leitenden Element (48), das die Innenseite des Handschuhs im wesentlichen ausfüllt und mit ihr in Kontakt ist und ein von dem Handschuh bedecktes leitendes Element bildet;
Zufuhr einer ionenhaltigen Flüssigkeit;
Verbinden eines ersten Endes einer ersten elektrischen Leitung (12) mit der ionenhaltigen Flüssigkeit und eines zweiten Endes der elektrischen Leitung mit einer elektrischen Spannungsquelle (24);

Eintauchen der Außenseite des Handschuhs in die Flüssigkeit, um das erste Ende der ersten elektrischen Leitung elektrisch mit der Außenseite des Handschuhs zu verbinden;
Verbinden eines ersten Endes einer zweiten elektrischen Leitung (14) mit dem leitenden Element und eines zweiten Endes der Leitung mit der Spannungsquelle;
Messen der elektrischen Eigenschaften zwischen dem ersten Ende der ersten Leitung und dem ersten Ende der zweiten Leitung mit einer elektrischen Meßeinrichtung (18, 20), die elektrisch mit der ersten und der zweiten Leitung verbunden ist, das der Feststellung eines Lochs in dem Handschuh während des Kontaktes der Außenseite desselben mit der Flüssigkeit dient, die durch Ionen in der Flüssigkeit bestimmt wird, die in das Loch eindringen und einen elektrischen Stromkreis zwischen der ersten und der zweiten Leitung schließen;
gekennzeichnet durch die Schritte der Zufuhr der ionenhaltigen Flüssigkeit in Form eines Flüssigkeitsstroms;
des Verbindens des ersten Endes der ersten elektrischen Leitung (12) mit dem Flüssigkeitsstrom; und
des Ableitens des Flüssigkeitsstroms ohne Rückführung nach dem Kontakt mit der Außenseite des Handschuhs.

5. Verfahren zur Feststellung eines Lochs in einem Operations- oder Untersuchungshandschuh, der aus einem im wesentlichen nichtleitenden Material besteht, nach Anspruch 4, wobei der Strom ionenhaltiger Flüssigkeit durch einen Strom von Leitungswasser aus einem offenen Hahn (42) gebildet wird.

6. Verfahren zur Feststellung eines Lochs in einem Operations- oder Untersuchungshandschuh, der aus einem im wesentlichen nichtleitenden Material besteht, nach Anspruch 4, wobei der Strom ionenhaltiger Flüssigkeit durch einen Strom keimfreier Kochsalzlösung aus einem Behälter gebildet wird.

**Revendications**

1. Appareil permettant de détecter des trous ou des perforations dans un gant chirurgical ou d'examen (46) en caoutchouc ou en matière plastique possédant une surface interne et une surface externe, cette dernière étant soumise à des contacts avec des agents viraux et des bactéries potentiellement infectieux pendant l'utilisation dudit gant, ledit appareil comprenant :

un premier fil électriquement conducteur (12) ayant une première et une deuxième extrémité, la première extrémité venant indirectement en contact avec la surface externe dudit gant (46) par l'intermédiaire d'un liquide (44) contenant des ions lorsque la surface externe dudit gant est mise en contact avec ledit liquide, la deuxième extrémité étant raccordée à une source d'alimentation électrique (24) ;

un deuxième fil électriquement conducteur (14) ayant une première extrémité destinée à venir directement en contact avec un élément conducteur (48) qui est conçu pour venir sensiblement en contact avec la surface interne dudit gant, et dont la deuxième extrémité est reliée à ladite source d'alimentation électrique;

un moyen (18, 20) servant à mesurer les propriétés électriques entre ledit premier fil (12) et ledit deuxième fil (14) lorsqu'une fuite à travers ledit gant permet à des ions contenus dans ledit liquide qui est en contact avec la surface externe dudit gant de pénétrer par ladite fuite et de parcourir complètement le circuit formé entre ledit premier fil et ledit deuxième fil via ledit liquide, ledit moyen de mesure étant électriquement connecté audit premier fil et audit deuxième fil ;

caractérisé en ce que l'appareil comprend un moyen (41) d'alimentation en liquide qui sert à fournir un courant non recirculant du liquide contenant des ions, ladite première extrémité du premier fil électriquement conducteur (12) étant connectée au moyen (41) d'alimentation en liquide.

2. Appareil permettant de détecter des trous ou des perforations dans un gant chirurgical ou d'examen en caoutchouc ou en matière plastique selon la revendication 1, où ledit moyen (41) d'alimentation en liquide fournit un écoulement d'eau du robinet qui définit ledit courant de liquide contenant des ions, et où un moyen (42) faisant fonction de robinet est fonctionnellement associé audit moyen d'alimentation en liquide afin de commander l'écoulement de ladite eau du robinet.

3. Appareil permettant de détecter des trous ou des perforations dans un gant chirurgical ou d'examen en caoutchouc ou en matière plastique selon la revendication 1, où ledit moyen d'alimentation en liquide comprend un moyen formant un conteneur destiné à recevoir et à fournir un écoulement de solution saline stérile qui définit ledit courant de liquide contenant des ions.

4. Procédé permettant de détecter une fuite à travers un gant chirurgical ou d'examen (46) formé d'une matière sensiblement non conductrice et possédant une surface interne et une surface externe, cette dernière surface étant soumise à des contacts avec des agents viraux et des bactéries potentiellement infectieux pendant l'utilisation dudit gant, ledit procédé comprenant les opérations suivantes :

placer ledit gant sur un élément conducteur (48) qui remplit sensiblement ledit gant et est sensiblement en contact avec la surface interne de celui-ci de manière à former un élément conducteur revêtu par le gant ;

fournir un liquide contenant des ions ;

connecter une première extrémité d'un premier fil électrique (12) au liquide contenant des ions et la deuxième extrémité dudit fil électrique à une source d'alimentation électrique (24) ;

immerger la surface externe dudit gant dans ledit liquide afin de coupler électriquement ladite première extrémité du premier fil électrique avec la surface externe dudit gant ;

connecter une première extrémité d'un deuxième fil électrique (14) audit élément conducteur et la deuxième extrémité dudit fil à ladite source électrique ;

observer les propriétés électriques existant entre la première extrémité dudit premier fil et la première extrémité dudit deuxième fil à l'aide d'un dispositif de mesure électrique (18, 20) électriquement connecté auxdits premier et deuxième fils afin de détecter une fuite à travers le gant pendant le contact de la surface externe de celui-ci avec le liquide, comme déterminé par des ions dudit liquide qui pénètrent par ladite fuite et parcourent complètement le circuit électrique formé entre lesdits premier et deuxième fils ;

caractérisé par les opérations suivantes :

fournir ledit liquide contenant des ions sous la forme d'un courant de liquide ;

connecter la première extrémité du premier fil électrique (12) au courant de liquide ; et

rejeter, sans le faire recirculer, le courant de liquide après le contact avec la surface extérieure dudit gant.

5. Procédé de détection d'une fuite à travers un gant chirurgical ou d'examen formé d'une matière sensiblement non conductrice selon la revendication 4, où le courant de liquide contenant des ions est fourni par un écoulement d'eau du robinet venant d'un robinet ouvert (42).

6. Procédé de détection d'une fuite à travers un gant chirurgical ou d'examen formé d'une matière sensiblement non conductrice selon la revendication 4, où le courant de liquide contenant des ions est fourni par un écoulement de solution saline stérile venant d'un conteneur.

Fig. 1

Fig. 2

Fig. 3